# EUROPEAN PATENT SPECIFICATION

(11) **EP 1 393 698 B1**
(45) Date of publication and mention of the grant of the patent: **30.03.2005**
(21) Application number: 03021905.9
(22) Date of filing: 17.01.1997
(51) Int. Cl.: A61F 5/08

(54) **Epidermal lifting mechanism**
Nasal epidermaler Hebemechanismus
Mécanisme de soulèvement épidermique s'appliquant sur le nez

(30) Priority: 10.05.1996 US 17258 P
(43) Date of publication of application: 03.03.2004
(62) Divisional of application: 97904815.4
(73) Proprietor: Beaudry, Wallace J., Elkhart Lake, WI 53020 (US)
(72) Inventor: Beaudry, Wallace J., Elkhart Lake, WI 53020 (US)
(74) Representative: Sparing, Rolf Klaus

(56) References cited:
- WO-A-94/23675
- WO-A-96/01093

## Description

The invention relates to an epidermal lifting mechanism in accordance with the preamble of claim 1.

WO 96 01 093 A1 describes an epidermal lifting mechanism comprising a strip of flexible material having a first side including an adhesive material. The strip of flexible material includes two resilient spring structures each being designed as resilient band. The strip further comprises a first end section, a second end section each having a round shape and a middle section having two oppositely disposed edges, wherein the first and the second end section are for arranging on two sides of a bridge of a human nose.

WO 94 23 675 A1 describes an epidermal lifting mechanism for use as a nasal dilator comprising a top material being coupled to a base material, wherein two resilient bands and two flexible strips of interface adhesive material are arranged between the top material and the base material. The base material comprises a layer of adhesive material which is covered by two release liners.

It is the object of the invention to provide an epidermal lifting mechanism, which permits a safe and persistent holding on an epidermal area.

The invention achieves this object for the epidermal lifting mechanism mentioned at the beginning by the characterizing features of claim 1.

With respect the features of the present invention as an epidermal lifting mechanism, the epidermal lifting mechanism may be generally described as comprising at least one strip of material having a first side and a second side, the strip further including a first end portion and a second end portion. Between the first side and the second side are preferably one or more layers of predetermined materials.

These layers of materials include without limitation, a silicone coated release liner, an adhesive system to adhere the epidermal lifting mechanism to the nose, a top layer of material, and a spring mechanism. Obviously, the release liner is removed prior to placing the epidermal lifting mechanism on the bridge of the nose. The adhesive system, just like the adhesive system for the dressing mechanism, can include a pressure sensitive hypo-allergenic acrylic or a hydrocolloid material but any suitable adhesive system may be used. The top layer of material can be either a non-woven material or a material with some stretch characteristics such as a three mil polyurethane film. The spring mechanism may comprise a polyester film (usually 2 mils to 8 mils in thickness but any suitable thickness range may be used, e.g., 1 - 15 mils would be suitable as an alternative thickness range but any thickness range can be used depending upon the desired use and durability) laminated to a spun bonded polyester material. The spun bonded polyester material may or may not be coated with a pressure sensitive adhesive. The spring mechanism may be a plurality of materials which are laminated together.

Although unitary, the mechanism has the following components: a pair of nose pods and a bridge section. The nose pods include an exposed adhesive surface which is bonded to the skin on the sides of the nose. The bridge section of the device has at least one fulcrum point, located at the bridge of the nose when it is applied to the bridge of a nose, and lies across the bridge of the nose.

However, it should also be noted that the present invention could be applied to simply one side of the nose with the bridge section of the device ending at the top of the bridge of the nose and being adhered thereto. Alternatively, the bridge section could simply be a strip of resilient or elastic material which is connected to the cheek of the wearer at one end by use of an adhesive material and the nose pod being connected to the side of the nasal passage at the other end.

It should be noted that it is preferable for the nose pods to include horseshoe shaped slits or cuts which are made in the top layer of the material through the adhesive layer which, when applied to the nose, allows the spring action to generate a uniform lifting force in a suction cup like manner while at the same time applying a shearing force to the adhesive itself due to the presence of the slit structures, rather than a lifting force thereby creating flexibility from the lift point to the adhesion point. By decreasing the lifting (peel) force on the adhesive, the stability of the bond between the adhesive and the skin is greatly increased and allows more flexibility of the dilator during facial movement. Thus the dilator will stay comfortably in place even during vigorous movement by the wearer; even when used in applications other than a nasal dilator.

A pair of flaps attached adjacent to the bridge section of the epidermal lifting mechanism create another pair of fulcrum points (fulcrum point 2) between the bridge of the nose (fulcrum point 1) and the adhesive material thereby increasing the dilation force of the outer epidermis of the nasal passages. The additional fulcrum points are accomplished by folding of the flaps adjacent to the bridge section underneath the epidermal lifting mechanism allowing the adhesive area of each flap to adhere to the bottom adhesive area of the bridge section of the epidermal lifting mechanism securing it in place. The flaps include perforations for ease of folding.

As discussed above, the pair of flaps create an additional fulcrum point. Further, when folded they provide a cushioned area for the bridge of the nose to cover the adhesive on the underside of the epidermal lifting mechanism so when applied for several hours and then removed discomfort to the skin tissue on the bridge of the nose is eliminated.

When the top and bottom spring laminates are laminated together and the epidermal lifting mechanism is applied to the nose, the bending of the multi-level springing increases the opening force to the nasal passages over a single level spring. Adding a layer of spring material on top of another layer of spring material creates a leaf spring action. Because there is a stretching force introduced into the top layer when bent over a fulcrum point, a stronger spring action is created as compared to a single layer spring of equal of thickness. Furthermore, bending over a fulcrum point or at multiple fulcrum points further improves the spring action.

Additionally, various pod configurations may be used to allow for flexibility of the bottom spring and/or to allow the pods to conform to the irregular surfaces of the nose or epidermal layer to which they are applied.

A key advantage of this mechanism is that anytime a person engages in physical activity that increases his or her heart rate, this mechanism allows for the delivery of more oxygen to the lungs. Further, the mechanism allows for more air to be effectively exhaled and thus both inhalation and exhalation are enhanced so overall breathing efficiency is enhanced.

Alternatively, this invention is useful when used in combination with a method for increasing the flow rate of gas through the nasal passages, the method comprising the steps of applying the epidermal lifting mechanism by bending the spring material over the bridge of the nose so that the adhesive material of the nose pods comes into positive contact with the sides of the nose and releasing the nose pods thus allowing the springs to mechanically lift the epidermal surface of the nose and increase the size of the nasal passage openings.

Figure 1 is a top plan view of a prior art nasal strip.

Figure 1A is a top plan view of the prior art nasal strip of Figure 1 including the flaps of the present invention.

Figure 2 is a side elevational view of a relaxed multi-level spring.

Figure 3 is a side elevational view of a tensioned multi-leveled spring bent over a fulcrum point.

Figure 4 is a side elevational view of the epidermal lifting mechanism showing its layered components.

Figure 5 is a schematic side elevational view of the epidermal lifting mechanism wherein the arrows depict the sheer force and peeling forces.

Figure 6 is a top plan view of an end portion of the epidermal lifting mechanism.

Figure 7 is a bottom plan view of the epidermal lifting mechanism.

Figure 8 is a side elevational view depicting the primary layers of the epidermal lifting mechanism.

Figure 9 is a top plan view of an alternative embodiment of the epidermal lifting mechanism.

Figure 10 is a top plan view of an alternative embodiment of the epidermal lifting mechanism.

Figure 11 is a top plan view of an alternative embodiment of the epidermal lifting mechanism.

Figure 12 is a top plan view of an alternative embodiment of the epidermal lifting mechanism.

Figure 13 is a top plan view of an alternative embodiment of the epidermal lifting mechanism.

Figure 14 is a top plan view of an alternative embodiment of the epidermal lifting mechanism.

Figure 15 is a side elevational view showing the epidermal lifting mechanism properly positioned on the bridge of the nose.

Figure 16 is a side elevational view showing the epidermal lifting mechanism improperly positioned too high on the bridge of the nose.

Figure 17 is a side elevational view showing the epidermal lifting mechanism improperly positioned too low on the bridge of the nose.

Figure 18 is an exploded view of the preferred embodiment of the present invention.

Although the disclosure hereof is detailed and exact to enable those skilled in the art to practice the invention, the physical embodiments herein disclosed merely exemplify the invention which may be embodied in other specific structure. While the preferred embodiment has been described, the details may be changed without departing from the invention, which is defined by the claims.

The invention comprises an epidermal lifting mechanism for providing a lifting force to a predetermined epidermal area, such as the bridge of the nose, to provide an increased flow rate of gas through the nasal passage and will be referred to generally as 10 in the following detailed description.

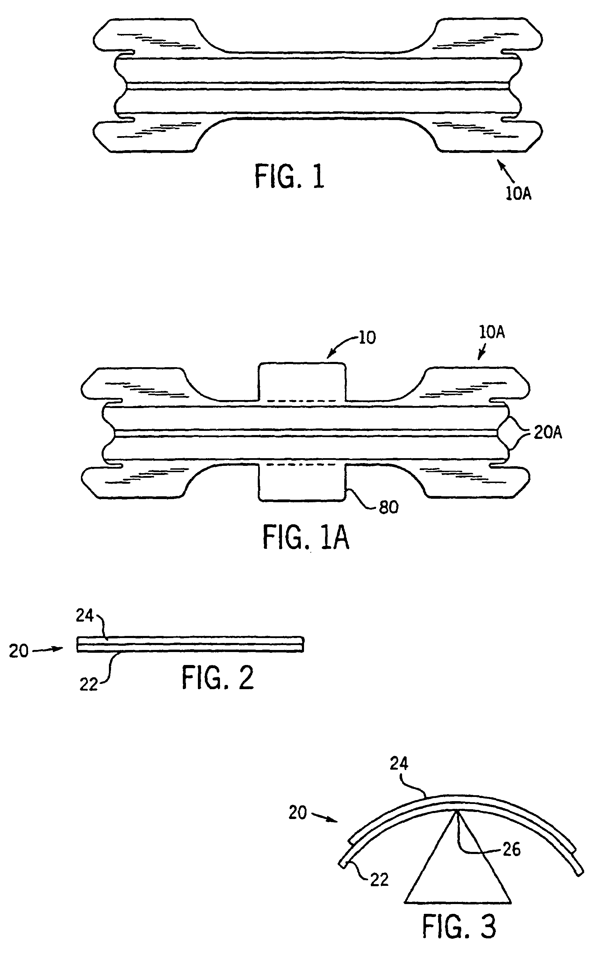

Referring to Figures 1 and 1A, a prior art device is shown. The prior art device shown in Figure 1 is currently marketed by CNS, Inc. of Chanhassen, Minnesota and sold under the trademark BREATHE RIGHT. The same device is shown in Figure 1A. however the device in Figure 1A includes the flaps of the present invention whose structure and advantages are discussed in detail below.

The present invention 10 includes a two part multi-level leaf spring 20 as shown in Figure 2. The two part multi-level leaf spring 20 comprises a pair of spring laminates 22 and 24. Each spring laminate 22 and 24 is manufactured from a 2 mil to 8 mil polyester film laminated to a spun bonded polyester material. The spun bonded polyester material may or may not be coated with a pressure sensitive adhesive. The spring laminates 22 and 24 are laminated together.

As illustrated in Figure 3, when the top 24 and bottom 22 spring laminates are laminated together and the invention 10 is applied to the bridge of the nose, represented by the fulcrum point 26, the bending of the multi-level spring 20 increases the opening force to the nasal passages over a single level spring.

Adding a layer of spring material 24 on top of another layer 22 of spring material creates a leaf spring action. Because there is a stretching force introduced into the top layer 24 when bent over a fulcrum point, a stronger (compound) spring action is created as compared to a single layer spring of equal thickness. Furthermore, bending over a fulcrum point creates a stronger yet spring action.

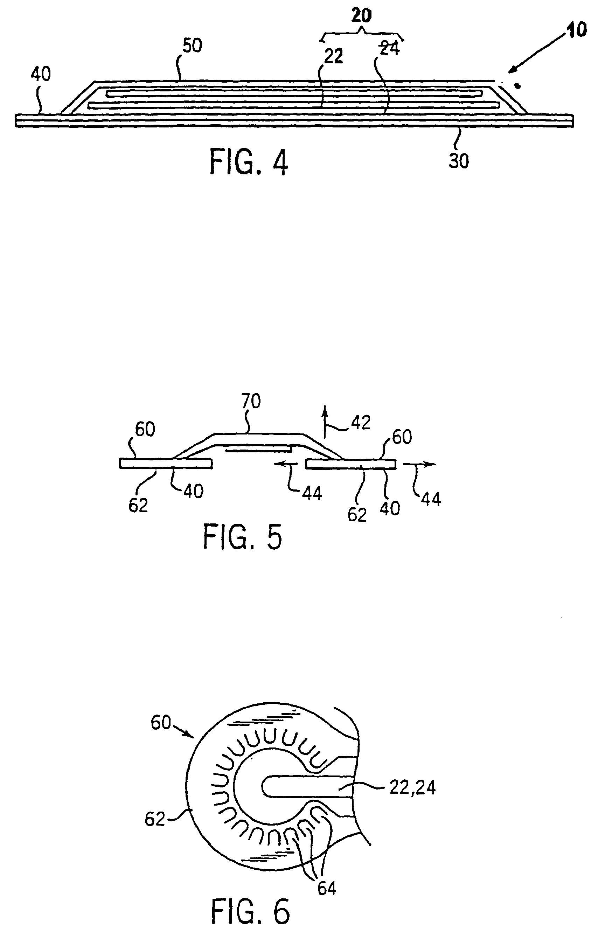

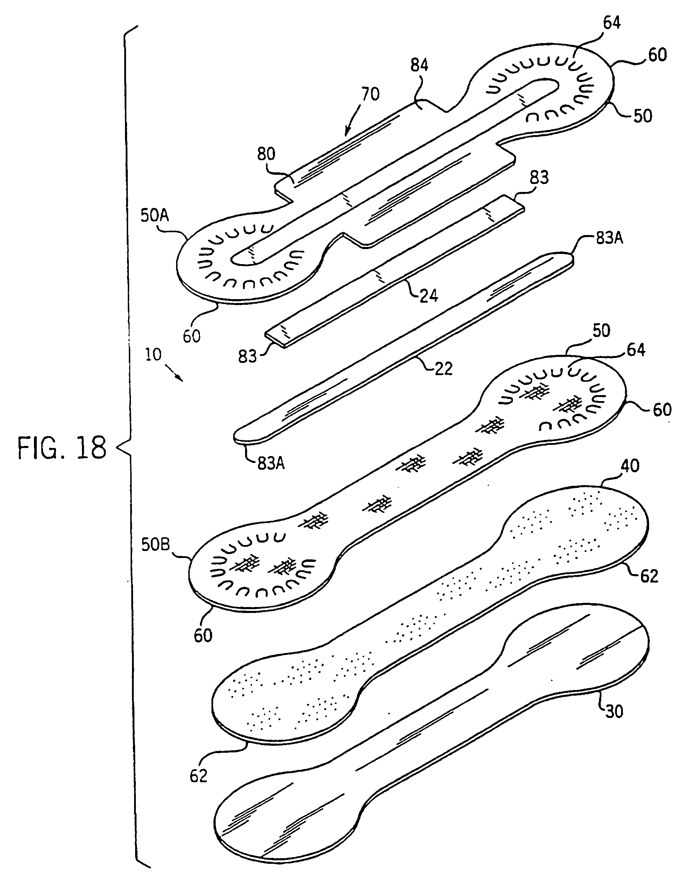

Now referring to Figures 4 and 18, the material layers of the invention 10 include a silicone coated release liner 30, an adhesive 40 to adhere the epidermal lifting mechanism 10 to the nose, a top layer of material 50, and the two part spring laminate 20. The top layer 50 is composed of two layers of material 50A and 50B and contains the springs 24 and 22 there between as shown in Figure 18. The release liner 30 is removed prior to placing the mechanism 10 on the bridge of the nose. The adhesive system 40 can either be a pressure sensitive hypo-allergenic acrylic or a hydrocolloid system. The top layer of material 50 can be either a non-woven material or a material with some stretch characteristics such as a 3 mil polyurethane film.

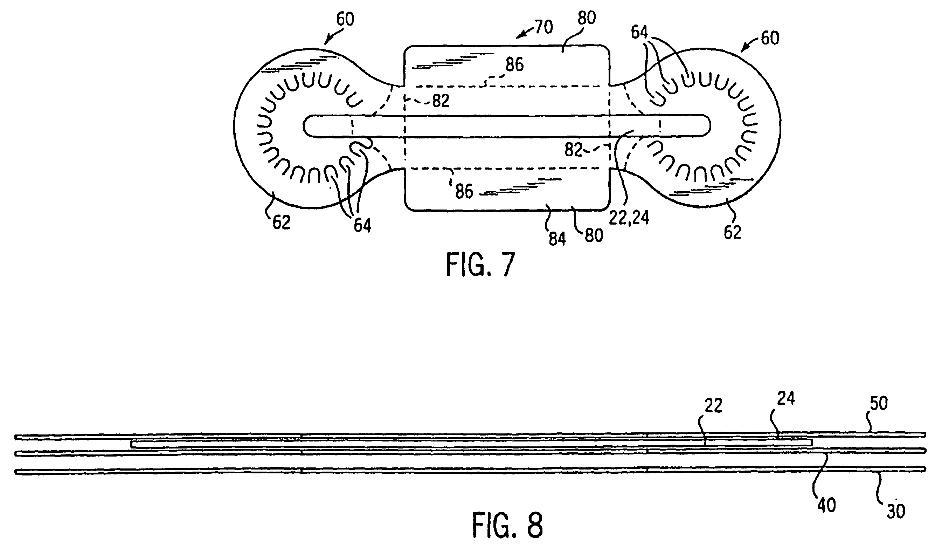

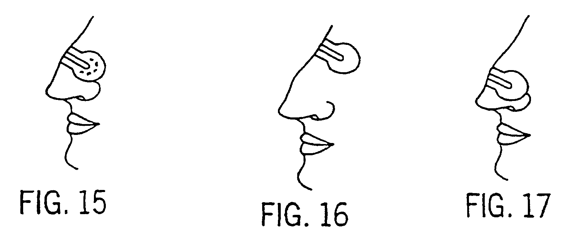

The preferred embodiment of the invention 10 is shown in Figure 7. Although unitary in construction, it has the following components: a pair of pods 60 and a bridge section 70. The pods 60 include an exposed adhesive surface 62 which is bonded to the skin on the sides of the nose. The pod 60 configurations allow for flexibility of the bottom spring 22 to conform to the irregular surfaces of the nose. The bridge section 70 of the device has at least one fulcrum point as shown in Figure 3 and lies across the bridge of the nose as shown in Figure 15.

As shown in Figure 6, the pods 60 include horseshoe shaped cuts or incisions 64 in the top layer of material 50 through the adhesive layer 40 which, when applied to the nose, allows the spring action to generate a uniform lifting force in a suction cuplike manner while at the same time applies a shearing force to the adhesive itself rather than a peeling force thereby creating flexibility from the lift point to the adhesion point. This principle is demonstrated in Figure 5. By decreasing the peel force 42 on the adhesive 40 the bond between the adhesive 40 and the skin is greatly increased and allows more flexibility of the epidermal lifting mechanism 10 during facial movement. The shearing forces are shown at 44.

Referring back to Figure 7, the contribution to the prior art by the present invention 10 is the inclusion of a pair of flaps 80 which are attached adjacent to the bridge section 70 of the invention 10. The flaps 80, when folded underneath or over the adhesive layer 40 of the bridge section 70, create another pair of fulcrum points along lines 82 between the bridge of the nose (fulcrum point 2) and the pods 60 when the invention 10 is applied to the wearer's nose. Thus, the flaps 80, when folded, function to increase the dilation force to the outer epidermis of the nasal passages.

More specifically, the additional fulcrum points 82 are accomplished by folding the flaps 80 underneath the bridge section 70 thereby allowing the adhesive area of each flap 84 to adhere to the bottom of the bridge section 70 thus securing it in place. The flaps 80 further include perforations 86 for ease of folding.

As discussed above, the pair of flaps 80 add fulcrum points. Accordingly, when the flaps 80 are folded they form end sections along lines 82 which will be located to either side of the bridge of the nose. Each of the end sections along lines 82 will act as a fulcrum point in addition to the bridge of the nose thereby increasing the number of fulcrum points and the mechanical lifting ability of the present invention. Further, when folded they provide a cushioned area for the bridge of the nose and cover the adhesive 40 on the underside of the bridge section 70 so when applied for several hours and then removed, discomfort to the skin tissue on the bridge of the nose is greatly reduced or eliminated since no adhesive has been in contact with the bridge of the nose due to the barrier created by the flaps.

The material layers of the invention 10 are shown in Figure 8. Again, the layers include a silicone coated release liner 30, an adhesive system 40 to adhere the epidermal lifting mechanism 10 to the nose, a first spring laminate 22, a second spring laminate 24, and a top layer of material 50.

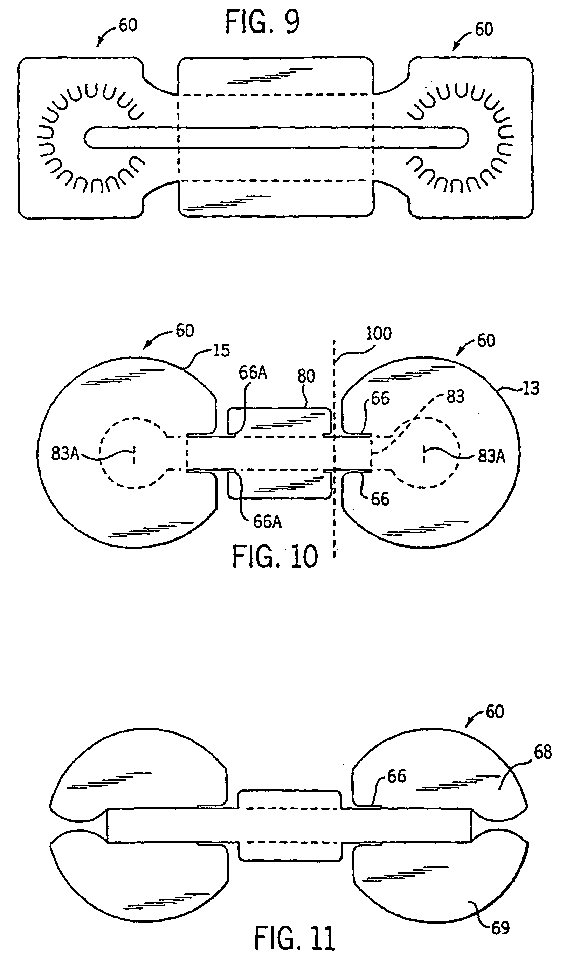

Alternative embodiments of the invention 10 are shown in Figures 9 through 14. In Figure 9, the shape of the pods 60 are shown to be rectangular instead of round. In Figure 10, the horseshoe shaped cuts or incisions 64 have been removed and additional slits 66 and 66A have been added. In this embodiment, when the flaps 80 are not folded over, slits 66A mechanically adjust the peeling action to a shear action thereby allowing greater adhesion over the predetermined epidermal area. Additionally, in this embodiment a cut could be made along line 100 to divide the invention 10 into sections 13 and 15 whereby section 13 could be discarded and section 15 could be used as a dilator for only one side of a persons nose. Additionally, two section 15's could be combined to apply to either side of a persons nose and thereby dilate each nasal passage independent of the other. Additionally, this embodiment includes springs 24 and 22 which are of different lengths as shown in the Figures 3 and 18. The ends of springs, shown by lines 83 and 83A, provide the main lifting force as the springs 24 and 22 attempt to spring back into position. Therefore, due to the mechanical relationship of springs 24 and 22 compound the lifting force applied at their ends 83 and 83A.

The embodiment shown in Figure 11 includes slits 66 and further includes a two-part pod 60. Pod 60 comprises an upper pod half 68 and a lower pod half 69. Pod halves 68 and 69 and slits 66 allow for greater flexibility of the pod 60 on the nose of the wearer.

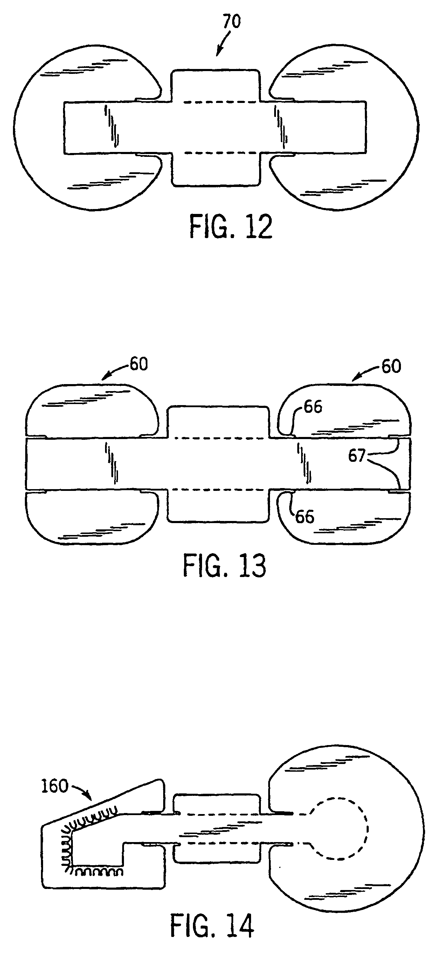

The embodiment shown in Figure 12 is similar to that shown in Figure 10 with the exception that the bridge section 70 has been widened. The embodiment of Figure 13 includes the wider bridge section 70 in combination with rectangular pods 60. Additional slits 67 have also been added near the outer sides of the pods 60. Slits 67 change the direction of the force applied to the pods 60 so that instead of a peel force (a force which tends to peel away the pods 60 from the epidermis to which they are applied) to a sheer forces (a force which tends to drag the pods 60 across the epidermis to which they are applied).

The embodiment depicted in Figure 14 demonstrates the principal that different pod 60 configurations can be used on the same epidermal lifting mechanism 10. The pod 60 shown on the left side has a sloping side to allow for better adhesion to the side of the nose.

The application of the invention 10 to the nose of the wearer is shown in Figures 15 through 17. Preferred installation of the epidermal lifting mechanism 10 on the bridge of the nose is shown in Figure 15 while in Figure 16, the epidermal lifting mechanism 10 is applied too high on the nose and is applied too low in Figure 17. However, while the positions shown in Figures 16 and 17 are not preferred they are functional since the structure of the present invention 10 allows a user the ability to apply the invention 10 over a relatively large epidermal area and thus effectiveness of the present invention is greatly enhanced. The present invention , will generally work effectively in all the positions shown in Figures 15-17.

Alternatively, this invention 10 could be used in combination with a method for increasing the flow rate of gas through the nasal passages, the method comprising the steps of removing the release liner 30, and positioning the invention 10 as shown in Figure 15 or as shown in Figures 16 and 17, depending upon the comfort of the wearer.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. An epidermal lifting mechanism for use and application to a nose of human being, providing a lifting force over an epidermal area located to either side of a bridge of said nose, the epidermal lifting mechanism comprising
at least one strip of flexible material (50) having a first side and a second side, the strip further including a first end portion (60), a second end portion (60) and a middle portion (70);
wherein the strip of flexible material (50) includes a plurality of resilient spring structures (20, 22, 24) extending therethrough;
wherein the first side of the strip includes an adhesive material (40);
wherein the first end portion (60) and the second end portion (60) each being capable of being positioned to cover a predetermined portion of an epidermis located adjacent each side of the bridge of the nose; and
wherein the middle portion (70) includes a first edge and second, oppositely disposed edge,
**characterized in**
**that** the first edge and the second edge of the middle portion (70) include an integral flap mechanism (80), and
**that** the flap mechanism (80) is capable of being folded over the first side of the strip of flexible material (50) to cover the middle portion (70) of the first side of the strip material (50).

2. The epidermal lifting mechanism according to claim 1, wherein the adhesive material (40) is a hydrocolloid non-irritating adhesive.

3. The epidermal lifting mechanism according to claim 1 or 2, wherein the epidermal lifting mechanism is adapted for use in combination with the bridge of said nose, the bridge of said nose providing a fulcrum point.

4. The epidermal lifting mechanism according to one of claims 1 to 3 wherein the first end portion (60) and the second end portion (60) have a plurality of slits (64) extending through at least the first side of the strip material (50).

5. The epidermal lifting mechanism according to claim 4, wherein the slits (64) have a U-shape.

6. The epidermal lifting mechanism according to one of claims 1 to 5, wherein the plurality of resilient spring structures (20, 22, 24) comprises a stack spring mechanism composed of a least two leaf spring structures (22, 24) stacked one on top of the other.

7. The epidermal lifting mechanism according to one of claims 1 to 6, wherein the flap mechanism (80) is folded over the first side and a fulcrum point is provided.

8. The epidermal lifting mechanism according to one of claims 1 to 7 wherein at least one of the first and second end portions (60) of the strip has a shape selected from the group comprising circular shape, rectangular shape, triangular shape.

9. The epidermal lifting mechanism according to one of claims 1 to 8 wherein the flap mechanism (80) comprises perforations (86) for ease of folding.

10. A method for applying an epidermal lifting mechanism according to one of claims 1 to 9 to a nose of human being, wherein before positioning the epidermal lifting mechanism to said nose, the flap mechanism (80) is folded back to the first side.

11. The method according to claim 10, wherein once the epidermal lifting mechanism is positioned an said nose, the plurality of resilient spring structures (20, 22, 24) urge either side of the nose such that the area effective for air flow through said nose is enlarged.

## Patentansprüche

1. Epidermische Hebevorrichtung für den Gebrauch und Anwendung an einer Nase eines Menschen, wobei der Hebemechanismus eine Hebekraft über einen epidermischen Bereich bereitstellt, die an den beiden Seiten des Nasenrückens vorgesehen ist, umfassend
wenigstens ein Band aus flexiblem Material (50), das wenigstens eine erste Seite und eine zweite Seite aufweist, wobei das Band ferner ein erstes Endteil (60), ein zweites Endteil (60) und ein mittleres Teil (70) umfasst;
wobei die erste Seite des Bandes ein selbstklebendes Material (40) umfasst;
wobei das erste Endteil (60) und das zweite Endteil (60) jeweils geeignet sind, um derart positioniert zu werden, um einen vorbestimmten Bereich der Haut, der angrenzend an jeder Seite des Nasenrückens vorgesehen ist, zu überdecken; und
wobei der mittlere Teil (70) eine erste Kante und eine zweite, gegenüberliegend angeordnete Kante aufweist,
**dadurch gekennzeichnet,**
**dass** die erste Kante und die zweite Kante des Mittelteils (70) einen integrierten Klappmechanismus (80) aufweisen, und
**dass** der Klappmechanismus (80) geeignet ist, um über die erste Seite des Bandes aus flexiblem Material (50) gefaltet zu werden, um das Mittelteil (70) der ersten Seite des Bandmaterials (50) zu überdecken.

2. Epidermale Hebevorrichtung nach Anspruch 1, wobei das selbstklebende Material (40) ein hydrocolloides nicht-irritierendes Klebematerial ist.

3. Epidermale Hebevorrichtung nach Anspruch 1 oder 2, wobei die epidermale Hebevorrichtung geeignet ist für den Gebrauch in Kombination mit dem Rücken der Nase, wobei durch den Rücken der Nase ein Stützpunkt gegeben ist.

4. Epidermale Hebevorrichtung nach einem der Ansprüche 1 bis 3, wobei das erste Endteil (60) und das zweite Endteil (60) eine Vielzahl von Schlitzen (64) aufweisen, wobei die Schlitze (60) sich wenigstens durch die erste Seite des Bandmaterials (50) erstrecken.

5. Epidermale Hebevorrchtung nach Anspruch 4, wobei die Schlitze (64) U-Form aufweisen.

6. Epidermale Hebevorrichtung nach einem der Ansprüche 1 bis 5, wobei eine Vielzahl von elastischen Federanordnungen (20, 22, 24) eine Stapelfedervorrichtung aufweisen, die wenigstens zwei Blattfederanordnungen (22, 24) umfassen, die aufeinander gestapelt sind.

7. Epidermale Hebevorrichtung nach einem der Ansprüche 1 bis 6, wobei der Klappmechanismus (80) über die erste Seite gefaltet ist und einen Stützpunkt gegeben ist.

8. Epidermale Hebevorrichtung nach einem der Ansprüche 1 bis 7, wobei wenigstens eine von dem ersten und dem zweiten Endteil (60) des Bandes eine von kreisförmig, rechteckig oder dreieckig ausgebildete Form aufweist.

9. Epidermale Hebevorrichtung nach einem der Ansprüche 1 bis 8, wobei der Klappmechanismus (80) Perforationen (86) für die Erleichterung des Faltens aufweist.

10. Ein Verfahren zur Anwendung der epidermalen Hebevorrichtung nach einem der Ansprüche 1 bis 9 an einer Nase eines Menschen, wobei vor dem Positionieren der epidermalen Hebevorrichtung an die Nase, der Klappmechanismus (60) auf die erste Seite zurückgefaltet wird.

11. Verfahren nach Anspruch 10, wobei sobald die epidermale Hebevorrichtung an der Nase positioniert wird, die Vielzahl von elastischen Federstrukturen (20, 22, 24) jede Seite der Nase derart drücken, dass der effektive Bereich für den Luftdurchfluss der Nase vergrößert ist.

## Revendications

1. Un mécanisme de soulèvement épidermique pour une utilisation et une application sur le nez d'un être humain, fournissant une force de soulèvement au-dessus d'une zone localisée d'un côté ou de l'autre d'un pont dudit nez, le mécanisme de soulèvement épidermique comprenant :
au moins une bande en matériau flexible (50) ayant un premier côté et un deuxième côté, la bande comprenant en outre une première partie d'extrémité (60) une deuxième partie d'extrémité (60) et une partie centrale (70) ;
dans lequel la bande en matériau flexible (50) comprend une pluralité de structures élastiques à ressort (20, 22, 24) s'étendant à travers celle-ci ;
dans lequel le premier côté de la bande comprend un matériau adhésif (40) ;
dans lequel la première partie d'extrémité (60) et la deuxième partie d'extrémité (60) étant chacune capable d'être positionnée de manière à recouvrir une partie prédéterminée d'un épiderme localisé de façon adjacente de chaque côté du pont du nez, et
dans lequel la partie centrale (70) comprend un premier bord et un deuxième bord disposé de façon opposée,
**caractérisé en ce que**
le premier bord et le deuxième bord de la partie centrale (70) comprennent un mécanisme de rabat intégral (80), et
**en ce que** le mécanisme de rabat (80) est pliable par dessus le premier côté de la bande en matériau flexible (50) pour recouvrir la partie centrale (70) du premier côté du matériau de bande (50).

2. Le mécanisme de soulèvement épidermique selon la revendication 1, dans lequel le matériau adhésif (40) est un adhésif non irritant hydrocolloïde.

3. Le mécanisme de soulèvement épidermique selon l'une des revendications 1 ou 2, dans lequel le mécanisme de soulèvement épidermique est adapté pour une utilisation en combinaison avec le pont dudit nez, le pont dudit nez fournissant un point d'appui.

4. Le mécanisme de soulèvement épidermique selon l'une des revendications 1 à 3, dans lequel la première partie d'extrémité (60) et la deuxième partie d'extrémité (60) ont une pluralité de fentes (64) s'étendant à travers au moins le premier côté du matériau en bande (50).

5. Le mécanisme de soulèvement épidermique selon la revendication 4, dans lequel les fentes (64) ont une forme en U.

6. Le mécanisme de soulèvement épidermique selon l'une des revendications 1 à 5, dans lequel la pluralité de structures élastiques à ressort (20, 22, 24) comprend un mécanisme de ressorts en pile composé d'au moins deux structures de ressort à lames (22, 24) empilées l'une sur l'autre.

7. Le mécanisme de soulèvement épidermique selon l'une des revendications 1 à 6, dans lequel le mécanisme de rabat (80) est plié au-dessus du premier côté et dans lequel un point d'appui est fourni.

8. Le mécanisme de soulèvement épidermique selon l'une des revendications 1 à 7, dans lequel au moins la première ou la deuxième partie d'extrémité (60) de la bande a une forme sélectionnée dans le groupe comprenant une forme circulaire, une forme rectangulaire, une forme triangulaire.

9. Le mécanisme de soulèvement épidermique selon l'une des revendications 1 à 8, dans lequel le mécanisme de rabat (80) comprend des perforations (86) pour faciliter le pliage.

10. Une méthode pour appliquer un mécanisme de soulèvement épidermique selon l'une des revendications 1 à 9 sur un nez d'un être humain, dans laquelle avant de positionner le mécanisme de soulèvement épidermique sur ledit nez, le mécanisme de rabat (80) est replié sur le premier côté.

11. La méthode selon la revendication 10, dans laquelle le mécanisme de soulèvement épidermique est positionné sur ledit nez, la pluralité de structures élastiques à ressort (20, 22, 24) lève l'un ou l'autre côté du nez de sorte que la zone efficace pour le flux d'air à travers ledit nez est agrandie.
